# EUROPEAN PATENT APPLICATION

(11) **EP 2 272 382 A1**
(43) Date of publication of application: **12.01.2011**
(21) Application number: 10176058.5
(22) Date of filing: 10.12.2002
(51) Int. Cl.: A23L 1/30, A61K 36/48, A61P 19/00

(54) **Composition for promotion of bone growth and maintenance of bone health, comprising soybean or soybean extracts**

(30) Priority: 11.12.2001 EP 01204838
(62) Divisional of application: 09172445.0
(71) Applicant: Société des Produits Nestlé S.A., 1800 Vevey (CH)
(72) Inventor: Offord-Cavin, Elizabeth, CH-1820 Montreux (CH); Federici, Ermanno, Oxford, OX3 7NP (GB); Lemaure, Bernard, F37000 Tours (FR); Courtois, Didier, F37550 St Avertin (FR)
(74) Representative: Brearley, Helen Rebecca

(57) **Abstract**

The present invention relates to a composition for maintenance of bone health or prevention, alleviation and/or treatment of bone disorders. It also relates to the use of the composition in the manufacture of a nutritional product, a supplement, a treat or a medicament; and a method of promoting bone growth or for the maintenance of bone health, which comprises administering an effective amount of the composition. Said composition contains a plant or plant extract containing phytochemicals, and the plant or plant extract is soya.

## Description

The present invention relates to a composition for maintenance of bone health or prevention, alleviation and/or treatment of bone disorders. It also relates to the use of the composition in the manufacture of a nutritional product, a supplement or a medicament; and a method of promoting bone growth or for the maintenance of bone health, which comprises administering an effective amount of the composition.

### Background of the Invention

Healthy bones require effective bone remodelling involving an equilibrium between bone formation and resorption. Most bone diseases are due to increased bone resorption, rendering its inhibition a primary therapeutic objective therefore most pharmaceutical agents, developed to date, are anti-resorptive. For example, estrogens block production of cytokines that promote osteoclast generation and differentiation. SERMs (selective estrogen response modulator) are being developed which provide benefits for bone health while reducing the risk of adverse hormonal effects on breast or endometrial tissues. It is assumed that they work by a similar mechanism to estrogen in bone. Bisphosphonates (such as alendronate, risedronate etc) concentrate in bone and are, to date, the most effective inhibitors of bone resorption. They inhibit a critical enzyme pathway, required for osteoclast activity and survival. Calcitonin is a polypeptide hormone that inhibits bone resorption by blocking osteoblast activity. New targets include blocking of the TNF receptor/ligand family members and their signalling pathways, particularly of RANK/RANKL, inhibition of bone-specific metalloproteinases such as cathepsin K or inhibition of specific masers.

Development of therapeutic agents stimulating bone formation has lagged behind that of resorption. Some chemical or pharmaceutical agents are known for promoting bone growth in human. For example, WO 9619246 describes a method for promoting bone growth in a human patient by intermittent administration of parathyroid hormone PTH-related protein or an agonist for at least one month. In WO 9619501, a pancreatic-derived factor inhibits the resorption of bone and stimulates bone cells to proliferate and increases the formation of bone.

A major recent breakthrough has been the demonstration of the role of bone morphogenic protein 2 (BMP-2) as a key player in stimulation of bone formation and that statins (effective drugs for cholesterol-lowering through inhibition of cholesterol synthesis) improve bone formation partly mediated by induction of BMP-2. The delivery of recombinant BMP-2 has been shown to induce bone or cartilage formation. In US 6150328, a method for the induction of bone and cartilage formation comprises administering a purified bone morphogenic protein produced by culturing a cell transformed with DNA encoding BMP, is described. Also, WO 9711095 relates to the use of bone morphogenic protein compositions for treatment of neural tumours and bone growth and wound healing. In addition to BMPs, growth factors such as insulin-like GF (IGF-1), transforming GF-β (TGF-β), fibroblast GFs (FGFs) are under investigation as local therapies in the healing of fractures and bone defects. However, systemic administration is problematic due to the metabolism in the intestine and also due to possible effects on other tissues. Gene therapy is being proposed as one option. The alternative is to target osteoblast regulation of their production by dietary or pharmaceutical regulators of their gene or protein expression.

Although these chemicals and pharmaceutical compounds have been proved for the treatment of bone disorders, it would be of interest to provide a safe, efficient nutritional way to promote bone growth and prevent, alleviate or treat bone/joint disorders in mammals.

### Summary of the Invention

Accordingly, in a first aspect, the present invention provides a composition intended for the prevention, alleviation and/or treatment of bone disorders or maintenance of bone health in mammals, which comprises as an active ingredient an effective amount of at least one plant or plant extract selected for its ability to induce bone morphogenic protein expression.

Remarkably, it has now been found that some plants or plant extracts have the ability to promote bone growth through regulation of endogenous growth factors in bone or cartilage tissue. They have the ability to induce bone morphogenic protein expression in vivo and on local bone environment, which has a positive effect on bone formation and repair, on maintenance of bone health or prevention, alleviation and/or treatment of bone disorders.

The composition according to the invention can be used in the manufacture of a nutritional product, a supplement, a treat or a medicament intended for humans or pets.

Administering to an individual a food composition according to the present invention, results in an improved bone regeneration during fracture healing. The present composition further helps to inhibit bone resorption. It helps to increase bone formation and bone mineral density during growth and optimize peak bone mass. Also, this food composition helps to decrease bone loss, in particular bone loss associated with age in mammals. It therefore helps to maintain bone mass with age and reduces the risk of osteoporosis.

Furthermore it helps to build cartilage in mammals, to prevent osteoarthritis in pets or humans, which results in a better activity or mobility of the individual.

In another aspect, the invention relates to the use of a plant or a plant extract selected for their ability to stimulate bone morphogenic proteins and/or inhibit bone resorption, for the preparation of a composition intended for the maintenance of bone health in mammals and for the prevention, alleviation and/or the treatment of bone disorders.

In addition the invention provides a method of prevention, alleviation and/or treatment of bone disorder or maintenance of bone health which comprises administering an effective amount of a composition as described above.

The invention further provides a method of increasing bone formation, bone mineral density during growth and optimize peak bone mass, treating or preventing osteoporosis, stimulating bone regeneration during fracture healing which comprises administering an elective amount of a composition as described above.

It further relates to a method for the treatment, alleviation and/or prophylaxis of osteoarthritis in pets and in humans, comprising the step of feeding an individual a composition as described above.

It further provides a method of decreasing bone loss, in particular bone loss associated with age in humans or pets, comprising the step of feeding the individual, a composition as described above.

### Detailed Description of the Invention

With respect to the first object of the present invention, the plant or plant extract according to the invention contains phytochemicals having an anabolic potential through induction of bone morphogenic protein expression and may further be anti-resorptive agents.

In a preferred embodiment, the plant or plant extract is from any part of the plant source, e.g. leaves, tubers, fruits, seeds, roots, grains, embryos or cell cultures. The plant or plant extract may be in the form of a dried, lyophilized extract of leaves, roots and/or fruits depending on the source of plant, or fresh plant, or enriched fraction obtained by inorganic or organic solvent extraction process known in the art.

The plant or plant extract is selected for its ability to inhibit bone resorption and/or induce bone formation, in particular it may be selected from the group consisting of *Lindera*, *Artemisia, Acorus, Carthamus, Carum, Cnidium, Amelanchier, Curcuma, Taraxacum, Cyperus, Juniperus, Prunus, Iris, Cichorium, Dodonaea, Epimedium, Eriogonum, Soya, Mentha, Ocimum, thymus, Tanacetum, Plantago, Spearmint, Bixa, Vitis, Rosemarinus, Tanacetum, Rhus* and *Anethum.* It may also be a mushroom

In a most preferred embodiment it may be aerial parts of *Lindera benzoin*, aerial part of *Artemisia vulgaris*, rhizome of *Acorus calamus,* seed or flower of *Carthamus tinctorius*, fruits of *Amelanchier ovalis*, fruits of *Amelanchier alnifolia,* roots of Cichorium intybus, rhizome of *Curcuma longa*, aerial part of *Epimedium brevicornum*, aerial part of *Eriogonum giganteum*, leaves or roots of *Taraxacum officinalis*, rhizome of *Cyperus rotondus*, leaves of *Dodoneae viscosa*, *Iris pallida* cell cultures, rhizome of *Iris germanica*, *pallida or pseudacorus*, fruit *of Juniperus communis,* seed of *Prunus persica*, soya cell cultures, aerial parts of *Mentha spicata*, aerial parts of *Ocimum gratissimum*, aerial parts of *Thymus* sp., aerial parts of *Rhus glabra*, aneth, *Bixa*, fruit of *Vitis vinifera*, aerial parts of *Rosmarinus officinalis*, aerial parts of *Tanacetum vulgare*, *Carum carvi*, *Plantago major*, aerial parts of *Oxydendron arboreum*, for example.

The phytochemicals may be genistein, daidzein, lactucin, lactucopicrin, 3-deoxy-lactucin, geraniol or carvone.

The plant or plant extract according to the Invention may be used in the preparation of a food composition. The said composition may be in the form of a nutritionally balanced food or pet food, a dietary supplement, a treat or a pharmaceutical composition.

The plant or plant extract may be used alone or in association with other plants such as chicory, tea, cocoa, or with other bioactive molecule such as antioxidants, fatty acids, prebiotic fibres, glucosamine, chondroitin sulphate, for example.

In one embodiment, a food composition for human consumption is prepared. This composition may be a nutritional complete formula, a dairy product, a chilled or shelf stable beverage, soup, a dietary supplement, a meal replacement, and a nutritional bar or a confectionery.

Apart from the plant extract according to the invention the nutritional formula may comprise a source of protein. Dietary proteins are preferably used as a source of protein. The dietary proteins may be any suitable dietary protein; for example animal proteins (such as milk proteins, meat proteins and egg proteins); vegetable proteins (such as soy protein, wheat protein, rice protein, and pea protein); mixtures of free amino acids; or combinations thereof. Milk proteins such as casein, whey proteins and soy proteins are particularly preferred. The composition may also contain a source of carbohydrates and a source of fat.

If the nutritional formula includes a fat source, the fat source preferably provides about 5% to about 55% of the energy of the nutritional formula; for example about 20% to about 50% of the energy. The lipids making up the fat source may be any suitable fat or fat mixtures. Vegetable fats are particularly suitable; for example soy oil, palm oil, coconut oil, safflower oil, sunflower oil, corn oil, canola oil, lecithins, and the like. Animal fats such as milk fats may also be added if desired.

A source of carbohydrate may be added to the nutritional formula. It preferably provides about 40% to about 80% of the energy of the nutritional composition. Any suitable carbohydrates may be used, for example sucrose lactose, glucose, fructose, corn syrup solids, and maltodextrins, and mixtures thereof. Dietary fibre may also be added if desired. If used, it preferably comprises up to about 5% of the energy of the nutritional formula. The dietary fibre may be from any suitable origin, including for example soy, pea, oat, pectin, guar gum, gum arabic, and fructooligosaccharides. Suitable vitamins and minerals may be included in the nutritional formula in an amount to meet the appropriate guidelines.

One or more food grade emulsifiers may be incorporated into the nutritional formula if desired; for example diacetyl tartaric acid esters of mono- and di- glycerides, lecithin and mono- and di-glycerides. Similarly suitable salts and stabilisers may be included. Vitamins and minerals may also be combined with the plant extract.

The nutritional composition is preferably enterally administrable; for example in the form of a powder, tablet capsule, a liquid concentrate, solid product or a ready-to-drink beverage. If it is desired to produce a powdered nutritional formula, the homogenised mixture is transferred to a suitable drying apparatus such as a spray drier or freeze drier and converted to powder.

In another embodiment, a nutritional composition comprises a milk-based cereal together with a prebiotic formulation. Preferably the milk-based cereal is an infant cereal which acts as a carrier for the prebiotic formulation.

In another embodiment, a usual food product may be enriched with at least one plant or plant extract according to the present invention. For example, a fermented milk, a yoghurt, a fresh cheese, a renneted milk, article of confectionery, for example a sweet or sweetened beverage, a confectionery bar, breakfast cereal flakes or bars, drinks, milk powders, soy-based products, non-milk fermented products or nutritional supplements for clinical nutrition.

The amount of the plant or plant extract in the composition may vary according to the plant source and its utilization. In a preferred embodiment, an efficient daily dose amount is of at least about 1 mg, and more preferably from 1 mg to 200mg of the active molecule per day.

In one embodiment, a pharmaceutical composition containing at least an extract or phytochemical as described above, in an amount sufficient to achieve the desired effect in an individual can be prepared. This composition may be a tablet, a liquid, capsules, soft capsules, pastes or pastilles, gums, or drinkable solutions or emulsions a dried oral supplement, a wet oral supplement. The pharmaceutical composition will further contain carriers and excipients that are suitable for delivering the respective active molecule of different nature to the target tissue. The kind of the carrier/excipient and the amount thereof will depend on the nature of the substance and the mode of drug delivery and/or administration contemplated. It will be appreciated that the skilled person will based on his own knowledge select the appropriate components and galenic form.

The plant or plant extract according to the invention may be used in the preparation of a pet food composition. The said composition may be administered to the pet as a supplement to its normal diet or as a component of a nutritionally complete pet food, and more preferably in an hypocaloric pet food. It may also be a pharmaceutical composition.

The plant or plant extract may be used alone or in association with other plants such as chicory, tea, cocoa, or with other bioactive molecule such as antioxidants, fatty acids, prebiotic fibers, glucosamine, chondroitin sulphate for example

Preferably, the pet food composition contains about 0.01 to 0.5 g of dry plants per gram of dry pet food for a 15 kg dog; and 0.001 to 0.1 g of dry plants per gram of wet pet food for a 15 kg dog.

The nutritionally complete pet food composition according to the invention may be in powdered, dried form a treat or a wet, chilled or shelf stable pet food product. It may be chilled or provided as a shelf stable product. These pet foods may be produced by ways known in the art.

The pet food may optionally also contain a prebiotic, a probiotic microorganism or another active agent, for example a long chain fatty acid. The amount of prebiotic in the pet food is preferably less than 10% by weight. For example, the prebiotic may comprise about 0.1% to about 5% by weight of the pet food. For pet foods which use chicory as the source of the prebiotic, the chicory may be included to comprise about 0.5% to about 10% by weight of the feed mixture; more preferably about 1% to about 5% by weight.

If a probiotic micro-organism is used, the pet food preferably contains about 10⁴ to about 10¹⁰ cells of the probiotic micro-organism per gram of the pet food; more preferably about 10⁶ to about 10⁸ cells of the probiotic micro-organism per gram. The pet food may contain about 0.5% to about 20% by weight of the mixture of the probiotic micro-organism; preferably about 1% to about 6% by weight; for example about 3% to about 6% by weight.

If necessary, the pet food is supplemented with minerals and vitamins so that they are nutritionally complete. Further, various other ingredients, for example, sugar, salt, spices, seasonings, flavouring agents, and the like may also be incorporated into the pet food as desired.

In another embodiment, dietary adjuncts may be prepared so as to improve pet food quality. As dietary adjuncts, they may be encapsulated or may be provided in powder form and packaged in conjunction with or separately from a main meal, be it wet or dry. By way of example, a powder containing extracts according to the invention, may be packed in sachets in a powder form or in a gel or lipid or other suitable carrier. These separately packaged units may be provided together with a main meal or in multi-unit packs for use with a main meal or treat, according to user instructions.

The amount of pet food to be consumed by the pet to obtain a beneficial effect will depend on the size of the pet, the type of pet, and age of the pet. However, an amount of the pet food to provide a daily amount of about 0.5 to 5 g of dry plants per kg of body weight, would usually be adequate for dogs and cats.

Administering to a human or animal, the food or pet food composition as described above, results in an improved bone regeneration during fracture healing. It helps to stimulate bone formation and bone mineral density during growth and optimize peak bone mass. In particular it may provide an optimal bone growth during childhood. This food composition helps to prevent bone loss, in particular bone loss associated with age in mammals or bone loss associated with long term hospitalization. It reduces risk of osteoporosis and improves recovery after fracture. Furthermore it helps to build cartilage in mammals, prevent osteoarthritis in pets and humans, which results in a better activity or mobility of the individual.

The following examples are given by way of illustration only and in no way should be construed as limiting the subject matter of the present application. All percentages are given by weight unless otherwise indicated. The examples are preceded by a brief description of the figure.

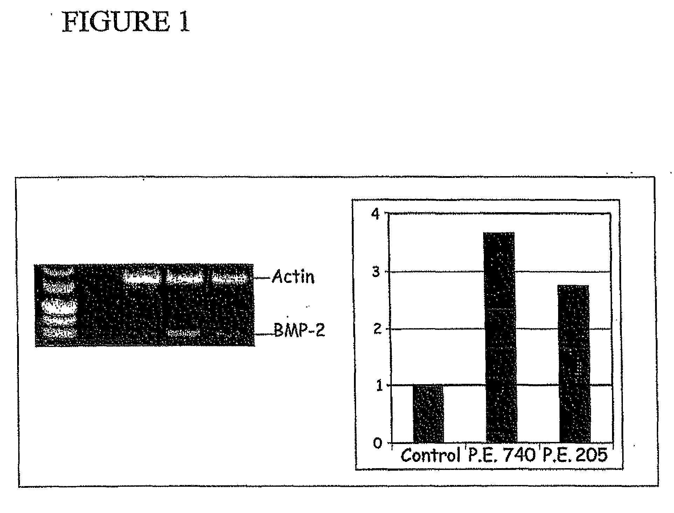

**Figure 1** **:** Measurement of endogenous BMP-2 mRNA expression in hPOB-tert cells by RT-PCR allowing treatment with extracts from *Lindera benzoin (P.E. 740,* 50 µg/ml)) *or Cyperus Rotundus (P.E. 205,* 10 µg/ml*)* for 48h and showing stimulation of BMP-2 by 3.8 and 2.8-fold of control, respectively. The validation of this assay has been performed with lovastatin (0.5 µg/ml) as a positive control showing induction of BMP-2 by 2.5 fold.

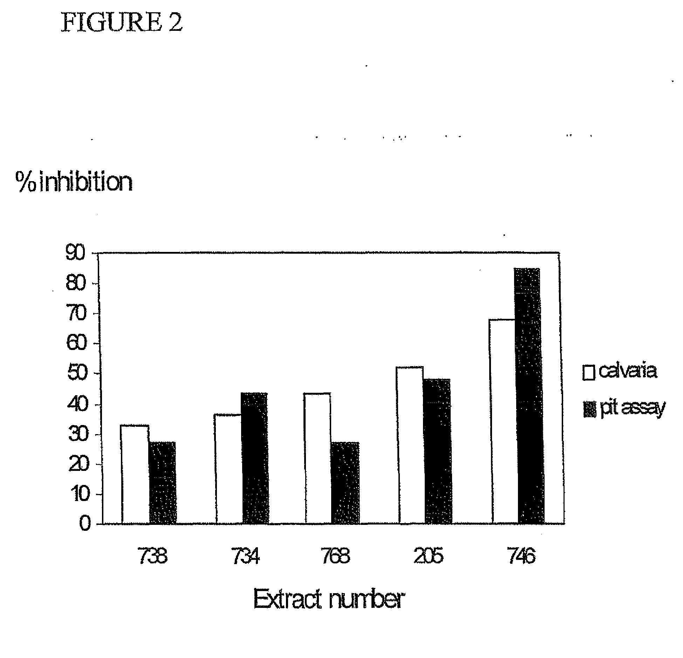

**Figure 2** **:** Comparison of measured inhibition values for the calvaria Assay (A) and the Pit Assay (B) for the extracts of *Ocimum gratissimum* (738), *Amelanchier alnifolia* (734), *Glycine max* (768), *Cyperus rotundus* (205), *Carthamus tinctorius* (746).

### Examples

### Example 1 : Assays on bone formation and bone resorption

### 1. borne formation

91 extracts were screened for bone formation through the BMP-2 (bone morphogenic protein) gene reporter assay and for bone resorption through the Calvaria assay. These 91 extracts correspond to 30 different plants.

### Materials and methods

### • Preparation of extracts for screening assays:

The ground plant material is defatted with hexane then extracted with a mixture of alcohol and water, with different percentages of water from 10 to 90%, preferably with 50%. The alcohols can be methyl or ethyl alcohols, giving the extract 1a.

On an aliquot of the residue of this first extract, an enzymatic hydrolysis is carried out with α and β glucosidases. Enzymes can be replaced by acidic conditions. The operation may be done under mild conditions (room temperature) or through reflux with different acid concentrations. The aqueous hydrolysed phase is extracted with a non-miscible solvent, preferably ethylacetate to give the extract 2a.

The extract can be dried, freeze-dried or supplied as a liquid form.
In some cases, polyphenols can be discarded through a polyvinylpolypyrrolidone (PVPP) treatment, avoiding artefact with the screening assays.

Following the extract preparation, each extract was weighed, redissolved in dimethylsulphoxide (DMSO) to a final concentration of 20 mg/ml and stored in aliquots at -20°C. This was used as a stock solution and was subsequently diluted in media for each assay. A range of doses was tested in the assay systems.

### • Bone formation assay

BMP-2 luciferase assay - The activity of the extracts was determined using 2T3 cells containing the BMP-2 promoter operatively linked to the luciferase gene. An increase in luciferase activity in cell lysates reflects an increase in BMP-2 promoter activity. The extracts were assayed at an initial dilution of 100 µg/ml with ½ dilutions down to 0.2 µg/ml. BMP-2 promoter activity was measured by measuring the luciferase activity in cell extracts.

13 plants gave a significant positive result in stimulation of BMP-2 expression (Table 1)

**Table 1**

| **Latin name** | **English name** | **part** | **conc** µ**g/ml** | **Active extract/n°** |
|---|---|---|---|---|
| *Acorus calamus* | sweet flag | rhizome | 5 | MeOH/water/731 |
| *Amelanchier ovalis* | service berry | fruit | 10 | MeOH/water/219 |
| *Artemisia vulgaris* | mugwort/w ormwood | aerial parts | 10 | Ethylacetate/225 |
| *Cyperus rotundus* | nutgrass | rhizome | 10 | Bthylacetate/205 |
| *Taraxacum officinalis* | common dandelion | leaves | 50 | ethylacetate/750 |
| *Lindera benzoin* | spice bush | aerial parts | 50 | ethylacetate/740 |
| *Prunus persica* | peach | seed | 25 | ethylacetate/772 |
| *Glycine max* | soybean | cell cultures | 50 | ethylacetate 768 |
| *Iris pallida* | sweet iris | tubers | 100 | MeOH/water/239 |
| *Rosmarinus officinalis* | rosemary | leaves | 50 | MeOH/water/2004 ethylacetate/2005 |
| *Carvi* | caraway | seeds | 25 | ethylacetate/2074 |
| *Thyme* | thyme | leaves | 25 | ethylacetate/2067 |
| *Mentha spicata* | mint | leaves | 100 | ethylacetate/2072 |
| *Vitis vinifera* | grape | fruit | 100 | ethylacetate/2069 |

Examples of new preparations of the same plants and subfractions thereof which stimulate BMP-2 are:
*Lindera benzoin,* active extract/n° ethylacetate/740/2059; Active subfraction/n° 2060 *Taraxacum officinalis,* active extract/n° ethylacetate/750/2034; Active subftaction/n° 2035
*Cyperus rotundus*, active extract/n° ethylacetate/205/2011; Active subfraction/n° 2012, 2013
*Iris pallida,* active extract/n° MeOH/water/239; Active subfraction/n° 760/762/2021, 2022
Subfractions were prepared by fractionation on reverse phase silica gel cartridge with elution by solvents of varying polarity. The pure soy isoflavones genistein and daidzein (10⁻⁶M) stimulated BWP-2 but estradiol did not.

BMP-2 induction seems to be not restricted to estrogenic-like activity, as it is stimulated by phytoestrogen but not by estrogen itself. It means that the activity of phytoestrogen (such as genistein and daidzein) may be mediated by a nonestrogenic mechanism. BMP-2 promoter activity is not stimulated by estradiol, thus estrogenicity of plant compounds is not required to be active in this test.

| Plant | Active extract no | concentration (µg/ml) |
|---|---|---|
| *Glycine max* | ethylacetate/2001 | 10, 50 |
| *Rosmarinus officinalis* | MeOH/water/2004 | 10, 50 |
| *Rosmarinus officinalis* | ethylacetate/2005 | 10 |
| *Cyperus rotundus* | subfraetion/2012 | 10, 50 |
| *Iris pallida* | subfraction/2022 | 10 |
| *Thyme* | ethylacetate/2067 | 10 |
| *Carvi* | ethylacetate/2074 | 10 |

Example of bone formation in calvaria organ culture

The method used is described in Science 286: 1946-1949 (1999). Extracts were assessed in a 4 day in vitro neonatal murine calvarial assay. Bones were incubated with the extracts for the entire 4 days. Bone formation was assessed by histology.

### 2. Bone resorption, calvaria assay

The ability of the extracts to inhibit IL-1 (10⁻¹⁰ M) stimulated bone resorption was assessed in the neonatal bone resorption assay. Each extract was assessed for its capacity to inhibit bone resorption at 10 µg/ml

The ability of the extracts prepared as in example 1, to inhibit IL-1 (10⁻¹⁰ M) stimulated bone resorption was assessed in the neonatal bone resorption assay, Each extract was assessed for its capacity to inhibit bone resorption at 10 µg/ml

The plant extracts found positive are listed in Table 2:

**Table 2.**

| **Latin name** | **English name** | **part** | **Active extract/n°** |
|---|---|---|---|
| *Amelanchier alnifolia* | serviceberry | fruit | ethylacetate/734 |
| *Ocimum gratissimum* | basil sp. | leaves | MoOH/H₂O/737 |
| *Ocimum gratissimum* | basil sp. | leaves | ethylacetate/738 |
| *Carthamus tinctorius* | safflower | seed | ethylacetate/746 |
| *Cyperus rotundus* | nutgrass | rhizome | ethylacetate/205 |
| *Glycine max* | soybean | cell cultures | 768 |

These plants were active in the bone resorption assay: *Amelanchier alnifolia, Ocimum gratissimum* and *Carthamus tinctorius and Glycine max. Cyperus rotundus* inhibited bone resorption and induced BMP-2.

### Example 2: Effect of plant extracts on endogenous BMP-2 expression in human osteoblast cells

The plants found positive for BMP-2 induction in example 1 were further tested in a human periosteal /preosteoblast cell line, hPOB-tert for their ability to induce the endogenous expression of BMP-2. This test in osteoblast cells confirmed the results shown in example 1.

For example, treatment of hPOB-tert cells with extracts of *Lindera benzoin* (extract 740, 50 µg/ml), and of *Cyperus rotundus* (extract 205, 10 µg/ml,) for 48h stimulated BMP-2 expression 3.8 and 2.8 fold of control (see Figure 1). The validation of this assay has been performed with lovastatin (0.5 µg/ml) as a positive control showing induction of BMP-2 by 2.5 fold.

At confluence, cells were incubated with 0.05 µg/ml Lovastatin or with the plant extract. Total RNA was extracted with TRIzol Reagent (Cribco). 10 µg RNA were reverse transcribed using the 1st Strand cDNA Syntheses Kit (Boehringer). BMP-2 cDNA sequences were amplified for 35 cycles at an annealing temperature of 55°C using specific oligonucleotide primers (5':TTGCGGCTGCTCAGCATGTT; 3':CATCTTGCATCTGTTCTCGGAA). PCR products were separated by agarose gel electrophoresis and detected by ethidium bromide staining. Quantification was performed using NIH image Software and normalizing results with Actin as housekeeping gene.

Results are shown in Figure 1.

### Bone resorption

The plant extracts found positive in the calvaria assay were retested in a second assay of bone resorption, namely in the pit assay using rabbit bone mixed cells cultured on bovine bone slices (Tezuka K., et al., 1992, Biochem. Biophys. Res. Commun. 186(2):911-7 and Lorget F., et al., 2000, Biochem. Biophys. Res. Commun. 268(3):899-903). Resorption pits were visualized by staining for TRAP (tartrate resistant acid phosphatase). Positive cells and counted.

A comparison of activity of the extracts at 10 µg/ml in the two assay systems is shown in Figure 2.

### Example 3: Dry pet food

A feed mixture is made up of about 58% by weight of corn, about 5.5% by weight of carn gluten, about 22% by weight of chicken meal, 2.5% dried chicory, about 10% of cyperus rotondus tubers, salts, vitamins and minerals making up the remainder.

The feed mixture is fed into a preconditioner and moistened. The moistened feed is then fed into an extruder-cooker and gelatinised. The gelatinised matrix leaving the extruder is forced through a die and extruded. The extrudate is cut into pieces suitable for feeding to dogs, dried at about 110°C for about 20 minutes, and cooled to form pellets.

This dry dog food has a positive effect on bone and cartilage health and increase their mobility.

### Example 4: Wet canned pet food with supplement

A mixture is prepared from 73 % of poultry carcass, pig lungs and beef liver (ground), 16 % of wheat flour, 2 % of dyes, vitamins, and inorganic salts. This mixture is emulsified at 12°C and extruded in the form of a pudding, which is then cooked at a temperature of 90°C. It is cooled to 30°C and cut in chunks. 45 % of these chunks are mixed with 55 % of a sauce prepared from 98 % of water, 1 % of dye, and 1 % of guar gum. Tinplate cans are filled and sterilised at 125°C for 40 min.

As a supplement to be mixed with the pet-food before serving, additional packaging (e.g. sachet) contains 25 g of powdered *Cyperus Rotundus* aerial parts to be added to the daily food. The corresponding amount for the pet is about 25 g / day and this can be supplied as a supplement with (e.g. on top of) the can.

In summary, described herein is:
1. A food composition intended for the prevention, alleviation and/or treatment of bone disorders and maintenance of bone health in humans and pets, which comprises as an active ingredient an effective amount of at least one plant or plant extract containing phytochemicals having the ability to induce bone morphogenic protein expression.
2. A composition according to 1, wherein the plant or plant extract further inhibits bone resorption.
3. A composition according to 1 or 2, wherein the plant is from any part of the plant source, leaves, tubers, fruits, seeds, roots, grains, embryos or cell cultures.
4. A composition according to one of 1 to 3, wherein the plant or plant extract is selected from the group comprising *Lindera, Artemisia, Acorus, Carthamus, Carum, Cnidium, Amelanchier, Curcuma, Taraxacum, Cyperus, Juniperus, Prunus, Iris, Cichorium, Dodonaea, Epimedium, Eriogonum, Soya, Mentha, Ocimum, thymus, Tanacetum, Plantago, Spearmint, Bixa, Vitis, Rosemarinus, Tanacetum, Rhus* and *Anethum.*
5. A composition according to one of 1 to 4, wherein the plant or plant extract is selected from aerial parts of *Lindera benzoin,* aerial part of *Artemisia vulgaris,* rhizome of *Acorus calamus,* seed or flower of *Carthamus tinctorius,* fruits of *Amelanchier ovalis,* fruits of *Amelanchier alnifolia,* roots of *Cichorium intybus,* rhizome of *Curcuma longa,* aerial part of *Epimedium brevicornum,* aerial part of *Eriogonum giganteum,* leaves or roots of *Taraxacum officinalis,* rhizome of *Cyperus rotondus,* leaves of *Dodoneae viscosa, Iris pallida* cell cultures, rhizome of *Iris germanica, pallida or pseudacorus,* fruit *of Juniperus communis,* seed of *Prunus persica,* soya cell cultures, aerial parts of *Mentha spicata,* aerial parts of *Ocimum gratissimum,* aerial parts of *Thymus sp.,* aerial parts of *Rhus glabra,* aneth, *Bixa,* fruit of *Vitis vinifera,* aerial parts of *Rosmarinus officinalis,* aerial parts of *Tanacetum vulgare, Carum carvi, Plantago major*, aerial parts of *Oxydendron arboreum.*
6. A composition according to one of 1 to 5, wherein the phytochemicals are lactucin, lactucopicrin, 3-deoxy-lactucin, peraniol or carvone.
7. A composition according to one of 1 to 6, which is in the form of a nutritionally balanced food or pet food, a dietary supplement, a treat or a pharmaceutical composition.
8. A composition according to one of 1 to 7, which helps bone regeneration during fracture healing, helps to increase bone formation and bone mineral density during growth and optimize peak bone mass or to decrease bone loss, in particular bone loss associated with age in humans or pets.
9. A composition according to one of 1 to 8, which helps to build cartilage in humans or pets.
10. A composition according to one of 1 to 9, which helps to prevent osteoarthritis in humans or pets, which results in a better activity or mobility of the individual.
11. Use of a plant or a plant extract containing phytochemicals having the ability to stimulate bone morphogenic protein and/or inhibit bone resorption for the preparation of a food or pet food composition intended for the prevention, the alleviation and/or the treatment of bone disorders or maintenance of bone health in humans or pets.
12. The use according to 11, wherein the composition is as described in 1 to 10.
13. The use according to 11 or 12, in which the plant or plant extract is used alone or in association with other plants such as chicory, tea, cocoa, or with other bioactive molecule such as antioxidants, fatty acids, prebiotic fibers, glucosamine, chondroitin sulphate for example
14. A method of treatment, alleviation or prevention of bone disorder or maintenance of bone health which comprises administering an effective amount of a composition according to any one of 1 to 6.
15. A method of increasing bone formation, bone mineral density during growth and optimize peak bone mass in humans or pets, comprising the step of feeding an individual, a composition according to any of 1 to 7.
16. A method for the treatment, alleviation and/or prophylaxis of osteoarthritis in pets and humans, comprising the step of feeding the individual a composition according to 1 to 7.
17. A method of treating or preventing osteoporosis, comprising administering an effective amount of a composition according to any one of 1 to 7.
18. A method of stimulating bone regeneration during fracture healing, comprising the step of feeding an individual, a composition according to any of 1 to 7.
19. A method of decreasing bone loss, in particular bone loss associated with age in humans or pets, comprising the step of feeding the individual, a composition according to any of 1 to 7.

## Claims

1. A food or pet food composition containing a plant or a plant extract containing phytochemicals having the ability to stimulate bone morphogenic protein and/or inhibit bone resorption, wherein the plant or plant extract is *Soya,* for the prevention, alleviation and/or treatment of bone disorders and maintenance of bone health in humans or pets.

2. A food or pet food composition according to claim 1, wherein the plant or plant extract further inhibits bone resorption.

3. A food or pet food composition according to claim 1 or 2, wherein the plant is from any part of the plant source, leaves, tubers, fruits, seeds, roots, grains, embryos or cell cultures.

4. A food or pet food composition according to one of claims 1 to 3, wherein the plant or plant extract is soya cell cultures.

5. A food or pet food composition according to one of claims 1 to 4, wherein the phytochemicals are lactucin, lactucopicrin, 3-deoxy-lactucin, geraniol or carvon.

6. A food or pet food composition according to one of claims 1 to 5, in which the plant or plant extract is used alone or in association with other plants such as chicory, tea, cocoa, or with other bioactive molecule such as antioxidants, fatty acids, prebiotic fibers, glucosamine, chondroitin sulphate for example.

7. A food or pet food composition according to one of claims 1 to 6, wherein the composition is in the form of a nutritionally balanced food or pet food, a dietary supplement, a treat or a pharmaceutical composition.

8. A food or pet food composition according to one of claims 1 to 7, wherein the composition helps bone regeneration during fracture healing, helps to increase bone formation and bone mineral density during growth and optimize peak bone mass or to decrease bone loss, in particular bone loss associated with age in humans or pets.

9. A food or pet food composition according to one of claims 1 to 7, wherein the composition helps to build cartilage in humans or pets.

10. A food or pet food composition according to one of claims 1 to 7, wherein the composition helps to prevent osteoarthritis in humans or pets, which results in a better activity or mobility of the individual.

11. A food or pet food composition according to one of claims 1 to 7, wherein the composition is for the treatment, alleviation and/or prophylaxis of osteoarthritis in pets and humans.

12. A food or pet food composition according to one of claims 1 to 7, wherein the composition is for treating or preventing osteoporosis.

13. A food or pet food composition according to one of claims 1 to 7, wherein the composition is for decreasing bone loss, in particular bone loss associated with age in humans or pets.
